# EUROPEAN PATENT SPECIFICATION

(11) **EP 4 518 773 B1**
(45) Date of publication and mention of the grant of the patent: **25.02.2026**
(21) Application number: 24726347.8
(22) Date of filing: 03.05.2024
(51) Int. Cl.: A61B 17/32, A61B 17/00

(54) **DISTAL NODE GROUNDING FEATURES FOR ACOUSTIC WAVEGUIDE**
ERDUNGSMERKMALE EINES DISTALEN KNOTENS FÜR AKUSTISCHEN WELLENLEITER
CARACTÉRISTIQUES DE MISE À LA TERRE DE NOEUD DISTAL POUR GUIDE D'ONDES ACOUSTIQUES

(30) Priority: 04.05.2023 US 202363463944 P
(43) Date of publication of application: 12.03.2025
(73) Proprietor: Cilag GmbH International, 6300 Zug (CH)
(72) Inventor: MONROE, David A., Cincinnati, Ohio 45242 (US); CLARK, Jeffrey L., Cincinnati, Ohio 45242 (US); HILL, Matthew T., Cincinnati, Ohio 45242 (US)
(74) Representative: Carpmaels & Ransford LLP
(86) International application number: PCT/IB2024/054312
(87) International publication number: WO 2024/228164

(56) References cited:
- WO-A1-2021/038375
- US-A1- 2015 320 437
- US-B1- 6 887 252

## Description

### PRIORITY

This application claims priority to U.S. Provisional Pat. App. No. 63/463,944, entitled "Articulating Acoustic Blade Distal Node Grounding," filed May 4, 2023.

### BACKGROUND

A variety of surgical instruments include an end effector having a blade element that vibrates at ultrasonic frequencies to cut and/or seal tissue (e.g., by denaturing proteins in tissue cells). These instruments include one or more piezoelectric elements that convert electrical power into ultrasonic vibrations, which are communicated along an acoustic waveguide to the blade element. The precision of cutting and coagulation may be controlled by the operator's technique and adjusting the power level, blade edge angle, tissue traction, blade pressure, and/or other conditions. The power level used to drive the blade element may be varied (e.g., in real time) based on sensed parameters such as tissue impedance, tissue temperature, tissue thickness, and/or other factors. Some ultrasonic instruments have a clamp arm and clamp pad for grasping tissue with the blade element.

Surgical instruments may be directly gripped and manipulated by a surgeon or incorporated into a robotic surgery system. During robotically assisted surgery, a surgeon may operate a controller to remotely control the motion of such surgical instruments at a surgical site. The controller may be separated from the patient by a significant distance (e.g., across the operating room, in a different room, or in a completely different building than the patient). Alternatively, a controller may be positioned quite near the patient in the operating room. The controller may include one or more hand input devices (such as joysticks, exoskeletal gloves, master manipulators, etc.), which are coupled by a servo mechanism to the surgical instrument. In some versions, a servo motor moves a manipulator supporting the surgical instrument based on the surgeon's manipulation of the hand input devices. During the surgery, the surgeon may employ, via a robotic surgical system, a variety of surgical instruments including an ultrasonic blade, a tissue grasper, a needle driver, an electrosurgical cautery probes, etc. Each of these structures performs functions for the surgeon, for example, cutting tissue, coagulating tissue, holding or driving a needle, grasping a blood vessel, dissecting tissue, or cauterizing tissue.

Examples of ultrasonic surgical instruments and related concepts are disclosed in U.S. Pat. No. 5,322,055, entitled "Clamp Coagulator/Cutting System for Ultrasonic Surgical Instruments," issued June 21, 1994; U.S. Pat. No. 6,325,811, entitled "Blades with Functional Balance Asymmetries for use with Ultrasonic Surgical Instruments," issued December 4, 2001; U.S. Pat. No. 6,773,444, entitled "Blades with Functional Balance Asymmetries for Use with Ultrasonic Surgical Instruments," issued August 10, 2004; U.S. Pat. No. 8,461,744, entitled "Rotating Transducer Mount for Ultrasonic Surgical Instruments," issued June 11, 2013; U.S. Pat. No. 8,591,536, entitled "Ultrasonic Surgical Instrument Blades," issued November 26, 2013; and U.S. Pat. No. 8,911,460, entitled "Ultrasonic Surgical Instruments," issued December 16, 2014.

Some ultrasonic surgical instruments may include an articulating shaft section. Examples of such ultrasonic surgical instruments are disclosed in U.S. Pat. No. 9,393,037, entitled "Surgical Instruments with Articulating Shafts," issued July 19, 2016; U.S. Pat. No. 9,095,367, entitled "Flexible Harmonic Waveguides/Blades for Surgical Instruments," issued August 4, 2015; U.S. Pat. No. 10,034,683, entitled "Ultrasonic Surgical Instrument with Rigidizing Articulation Drive Members," issued July 31, 2018; U.S. Pat. No. 10,405,876, entitled "Articulation Joint for Surgical Instrument," issued August 21, 2019; and U.S. Pat. No. 11,678,903, entitled "Ultrasonic Surgical Instrument with Articulating End Effector having a Curved Blade," issued May 31, 2023.

Examples of robotic surgical systems and instruments that may be used in such systems are described in U.S. Pat. No. 8,820,605, entitled "Robotically-Controlled Surgical Instruments," issued September 2, 2014; U.S. Pat. No. 9,301,759, entitled "Robotically-Controlled Surgical Instrument with Selectively Articulatable End Effector," issued April 5, 2016; U.S. Pat. No. 8,783,541, entitled "Robotically-Controlled Surgical End Effector System," issued July 22, 2014; U.S. Pat. No. 8,479,969, entitled "Drive Interface for Operably Coupling a Manipulatable Surgical Tool to a Robot," issued July 9, 2013; U.S. Pat. No. 8,800,838, entitled "Robotically-Controlled Cable-Based Surgical End Effectors," issued August 12, 2014; U.S. Pat. No. 10,166,082, entitled "System and Method for Controlling a Robotic Wrist," issued January 1, 2019; and U.S. Pat. No. 11,457,945, entitled "Ultrasonic Blade and Clamp Arm Alignment Features," issued October 4, 2022.

While several surgical instruments and systems have been made and used, it is believed that no one prior to the inventors has made or used the invention described in the appended claims.

US 6 887 252 B1 refers to an ultrasonic treatment appliance including a handpiece with a built-in ultrasonic transducer, and an elongated probe extending forward from the handpiece and connected to the ultrasonic transducer in order to propagate ultrasonic vibrations. The ultrasonic treatment appliance further comprises a distal probe part formed as an integral part of or independently of the probe at the distal end of the probe, and a clamping portion opposed to the distal probe part so that the clamping portion can open or close freely, the clamping portion being freely attachable thereto or detachable therefrom.

US 2015/320437 A1 refers to an apparatus comprising a body assembly, a shaft, an acoustic waveguide, an articulation section, an end effector, and an articulation drive assembly. The articulation section comprises a plurality of body portions aligned along the longitudinal axis and a flexible locking member. The flexible locking member is operable to secure the body portions in relation to each other and in relation to the shaft.

WO 2021/038375 A1 refers to a surgical instrument including an end effector, a shaft assembly, and an axial location feature. The shaft assembly includes a proximal shaft portion, an acoustic waveguide extending proximally from the ultrasonic blade, a distal shaft portion extending along a distal axis, and an articulation section interposed between the proximal shaft portion and the distal shaft portion. The axial location feature can inhibit the ultrasonic blade from shifting relative to the clamp arm along the distal axis as the end effector is driven between a non-deflected position and a deflected position.

### SUMMARY

The ultrasonic surgical instrument according to the invention is defined in claim 1. The method according to the invention is defined in claim 15. Embodiments of the invention are defined in the dependent claims.

### BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:
FIG. 1 depicts a first perspective view of an example of ultrasonic surgical instrument having an end effector, a shaft assembly, and a base assembly configured to connect to a robotic driven interface;
FIG. 2 depicts a second perspective view of the ultrasonic surgical instrument of FIG. 1;
FIG. 3A depicts an enlarged perspective view of a distal portion of the ultrasonic surgical instrument of FIG. 1, with the end effector in an open configuration and the shaft assembly in a straight configuration;
FIG. 3B depicts an enlarged perspective view of the distal portion of FIG. 3A, with the end effector in a closed configuration and the shaft assembly in the straight configuration;
FIG. 4 depicts a top plan view of the distal portion of FIG. 3A, with the end effector in the open configuration and the shaft assembly in the straight configuration;
FIG. 5 depicts a cross-sectional side view of the distal portion of FIG. 3A, with the end effector in the open configuration and the shaft assembly in the straight configuration;
FIG. 6 depicts a partially exploded perspective view of the distal portion of FIG. 3A, with a clamp arm of the end effector and a closure sleeve separated from other components of the shaft assembly;
FIG. 7 depicts another partially exploded perspective view of the distal portion of FIG. 3A, with the clamp arm and closure sleeve omitted, with an acoustic waveguide separated from other components of the shaft assembly, and with an elastomeric sleeve separated from other components of the shaft assembly;
FIG. 8 depicts a perspective view of a frame sleeve of the ultrasonic surgical instrument of FIG. 1;
FIG. 9 depicts a perspective view of a distal portion of the acoustic waveguide of the ultrasonic surgical instrument of FIG. 1;
FIG. 10 depicts a side elevation view of a distal node portion of the acoustic waveguide of FIG. 9;
FIG. 11 depicts a cross-sectional view of the acoustic waveguide of FIG. 9, taken along line 11-11 of FIG. 10;
FIG. 12 depicts a perspective view of a distal portion of another example of an acoustic waveguide that may be incorporated into the ultrasonic surgical instrument of FIG. 1;
FIG. 13 depicts a side elevation view of a distal node portion of the acoustic waveguide of FIG. 12;
FIG. 14 depicts a cross-sectional view of the acoustic waveguide of FIG. 12, taken along line 14-14 of FIG. 13;
FIG. 15 depicts a perspective view of a distal portion of another example of an acoustic waveguide that may be incorporated into the ultrasonic surgical instrument of FIG. 1;
FIG. 16 depicts a perspective view of a distal portion of another example of an acoustic waveguide, elastomeric sleeve, and crimping collet that may be incorporated into the ultrasonic surgical instrument of FIG. 1;
FIG. 17 depicts a perspective view of the crimping collet of FIG. 16; and
FIG. 18 depicts a perspective cross-sectional view of the assembly of FIG. 16 with an additional outer sleeve.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description serve to explain the principles of the technology; it being understood, however, that this technology is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following des cription, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of or dinary skill in the art in view of the teachings herein.

For clarity of disclosure, the terms "proximal" and "distal" are defined herein relative to a human or robotic operator of the surgical instrument. The term "proximal" refers the position of an element closer to the human or robotic operator of the surgical instrument and further away from the surgical end effector of the surgical instrument. The term "distal" refers to the position of an element closer to the surgical end effector of the surgical instrument and further away from the human or robotic operator of the surgical instrument. It will be further appreciated that, for convenience and clarity, spatial terms such as "front," "rear," "clockwise," "counterclockwise," "longitudinal," and "transverse" also are used herein for reference to relative positions and directions. Such terms are used below with reference to views as illustrated for clarity and are not intended to limit the invention described herein.

### I. Example of Ultrasonic Surgical Instrument

### A. Overview

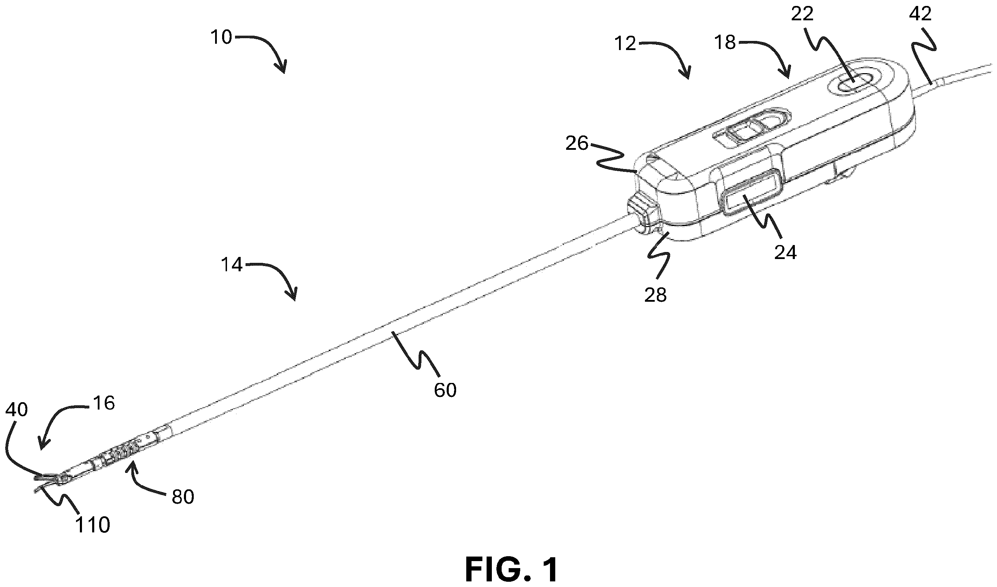

FIGS. 1-2 show an example of an ultrasonic surgical instrument (10). At least part of ultrasonic surgical instrument (10) may be constructed and operable in accordance with at least some of the teachings of any of the various patents that are cited herein. As described therein and as will be described in greater detail below, ultrasonic surgical instrument (10) is operable to cut tissue and seal or weld tissue (e.g., a blood vessel, etc.) substantially simultaneously. While the present example incorporates various ultrasonic features as ultrasonic surgical instrument (10), the invention is not intended to be unnecessarily limited to the ultrasonic features described herein.

Ultrasonic surgical instrument (10) of the present example comprises a body assembly, such as a base assembly (12), a shaft assembly (14), and an end effector (16). Base assembly (12) includes a housing (18), a button (22), and a pair of latch clasps (24). Button (22) is operatively connected to an electrical base power controller (not shown) and configured to selectively power ultrasonic surgical instrument (10) for use. In addition, housing (18) of the present example includes a front housing cover (26) and a rear housing cover (28) removably secured together via latch clasps (24). More particularly, latch clasps (24) removably secure front housing cover (26) to rear housing cover (28) such that front housing cover (26) may be removed for accessing an interior space (not shown) within base assembly (12).

Shaft assembly (14) distally extends from base assembly (12) to end effector (16). As shown in the present example, base assembly (12) is configured to operatively connect to a robotic drive (not shown) for driving various features of shaft assembly (14) and/or end effector (16). However, in another example, body assembly (12) may alternatively include a handle assembly (not shown), which may include a pistol grip and/or other structural features, configured to be directly gripped and manipulated by the surgeon for driving various features of shaft assembly (14) and/or end effector (16). The invention is thus not intended to be unnecessarily limited to use with base assembly (12) and the robotic drive (not shown). In other words, some variations of ultrasonic surgical instrument (10) may be hand held and hand manipulated, some variations of ultrasonic surgical instrument (10) may be robotically supported robotically manipulated, and still other variations may be subject to a combination of holding/supporting by hand and robot and/or manipulation by hand and robot.

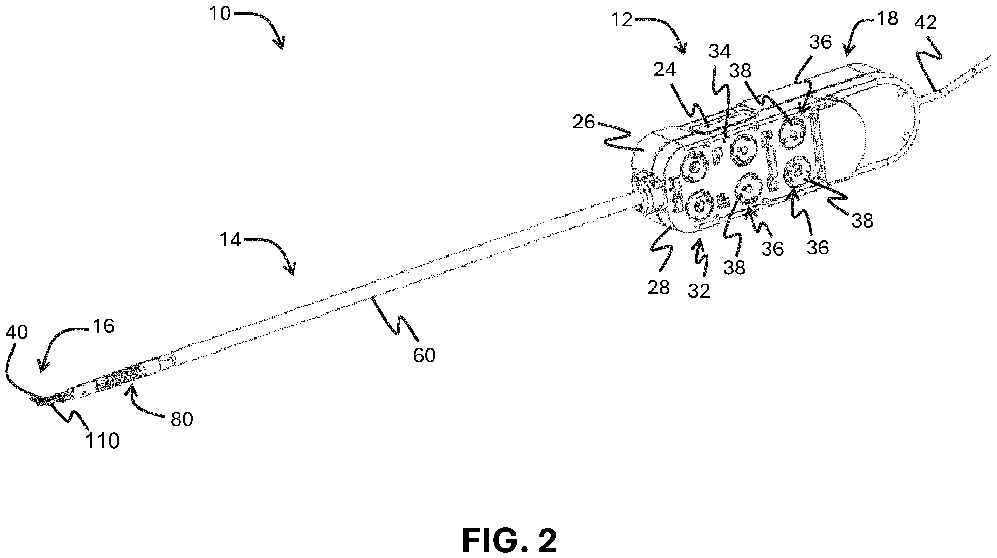

As shown in FIG. 2, base assembly (12) of the present example includes a robotic driven interface (32) extending through a base plate (34) of rear housing cover (28) and configured to mechanically couple with the robotic drive (not shown). Robotic driven interface (32) of the present example includes a plurality of instrument actuators (36) having a plurality of respective input bodies (38). Each input body (38), which may also be referred to herein as a "puck," is configured to removably connect with the robotic drive and, in the present example, is generally cylindrical and rotatable about a respective axis. Input bodies (38) have a plurality of slots and/or other features configured to engage corresponding portions of the robotic drive to thereby provide robotic driving of input bodies (38) to thereby direct operation of shaft assembly (14) and/or end effector (16).

Base assembly (12) of the present example also includes an electrical cable (42) that is operatively connected to an electrical power source (not shown) to provide electrical power to base assembly (12) for operation as desired, such as powering electrical base power controller (not shown) and directing electrical energy to various features of shaft assembly (14) or end effector (16) associated with cutting, sealing, or welding tissue. In some versions, an ultrasonic transducer (not shown) is contained within base assembly (12); and electrical power that is provided via cable (42) activates the ultrasonic transducer to thereby generate ultrasonic vibrations. The ultrasonic vibrations generated by the transducer may be communicated to end effector (16) as described below.

### B. Example of Shaft Assembly

As shown in FIGS. 1-4, shaft assembly (14) of the present example includes a proximal shaft portion (60) extending directly from base assembly (12) along a longitudinal axis (LA). A distal shaft portion (62) is fixedly coupled with proximal shaft portion (60). An articulation section (80) is coupled with distal shaft portion (62). Articulation section (80) includes a plurality of segments (82). Each segment (82) includes a proximally oriented tab (84) and a distally facing recess (86). Each tab (84) of each segment is pivotally secured within a corresponding recess (86), such that segments (82) are operable to pivot relative to each other. The tab (84) of the proximal-most segment (82) is pivotally coupled with distal shaft portion (62). A frame sleeve (200) is positioned distal to articulation section (80) and includes a proximally oriented tab (207) that is pivotally secured within the recess (86) of the distal-most segment (82).

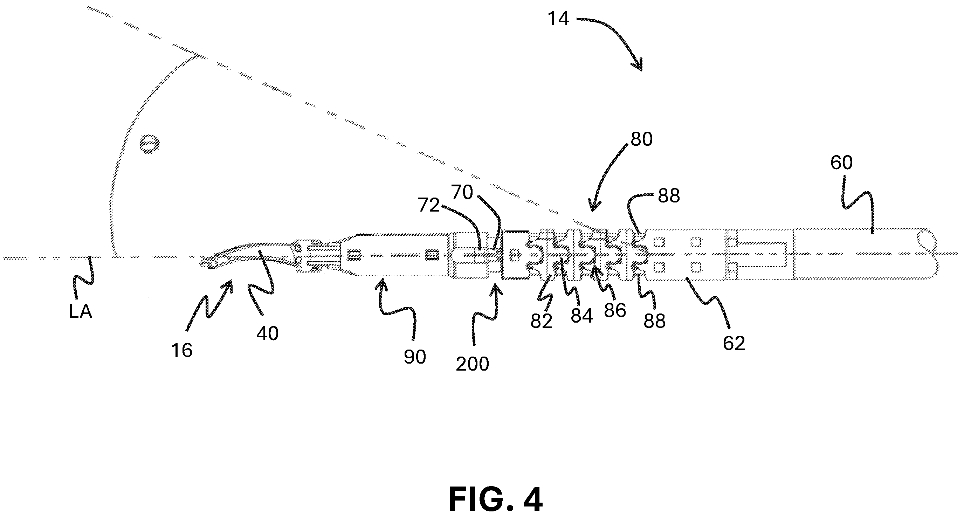

The pivotal couplings along articulation section (80) and at each end of articulation section (80) allow lateral deflection of end effector (16), relative to the longitudinal axis (LA), to thereby position end effector (16) at a selected articulation angle (e) as shown in FIG. 4. Such articulation movement is driven by articulation bands (88), which extend through segments (82) and are distally anchored in a proximal portion (208) of frame sleeve (200). Articulation bands (88) are angularly offset from each other by 180° about the longitudinal axis (LA). A first articulation band (88) may be advanced distally while a second articulation band (88) is retracted proximally, to thereby articulate end effector (16) in a first lateral direction; or the first articulation band (88) may be retracted proximally while the second articulation band (88) is advanced distally, to thereby articulate end effector (16) in a second lateral direction. Articulation bands (88) may be coupled with corresponding actuation features within base assembly (12) to thereby drive such opposing longitudinal movement of articulation bands (88). Such actuation features may be driven via corresponding input bodies (38) as described above.

By way of further example only, articulation section (80) may alternatively or additionally be configured in accordance with one or more teachings of U.S. Pat. No. 9,402,682, entitled "Articulation Joint Features for Articulating Surgical Device," issued August 2, 2016; U.S. Pat. No. 9,393,037, issued July 19, 2016, entitled "Surgical Instruments with Articulating Shafts,"; U.S. Pat. No. 9,095,367, issued August 4, 2015, entitled "Flexible Harmonic Waveguides/Blades for Surgical Instruments,"; and/or any other patent cited herein. Alternatively, articulation section (80) may be constructed and/or operable in any other suitable fashion.

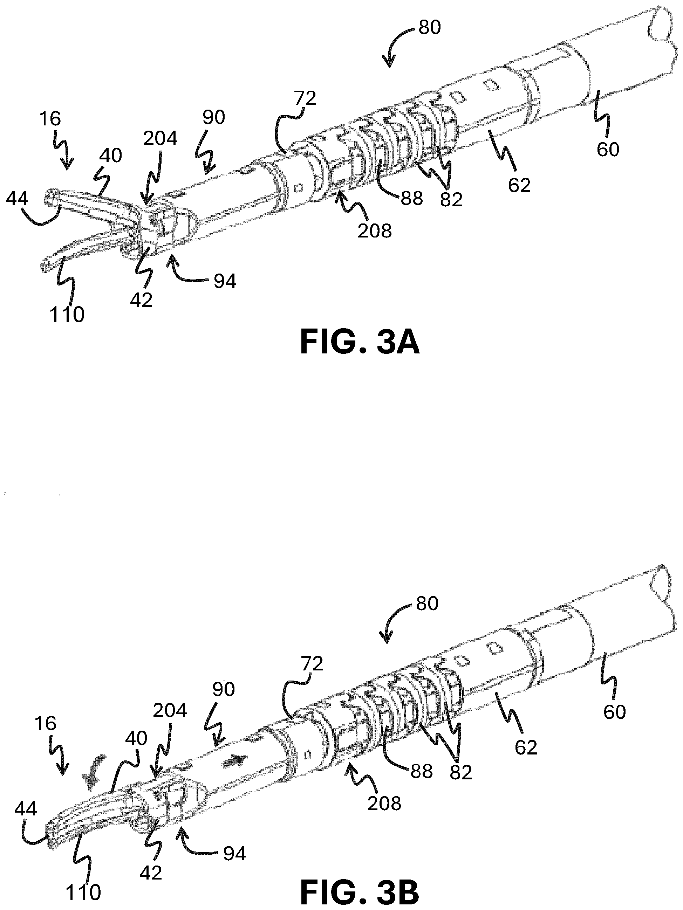

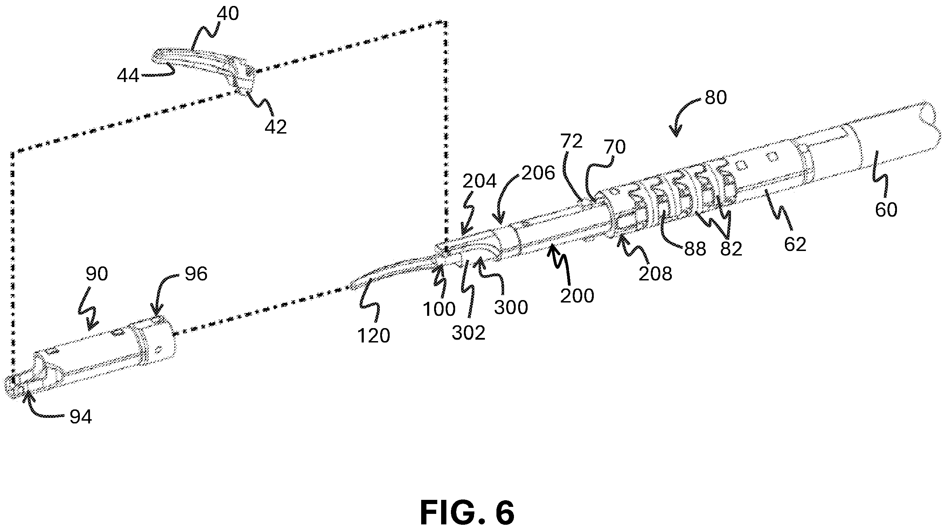

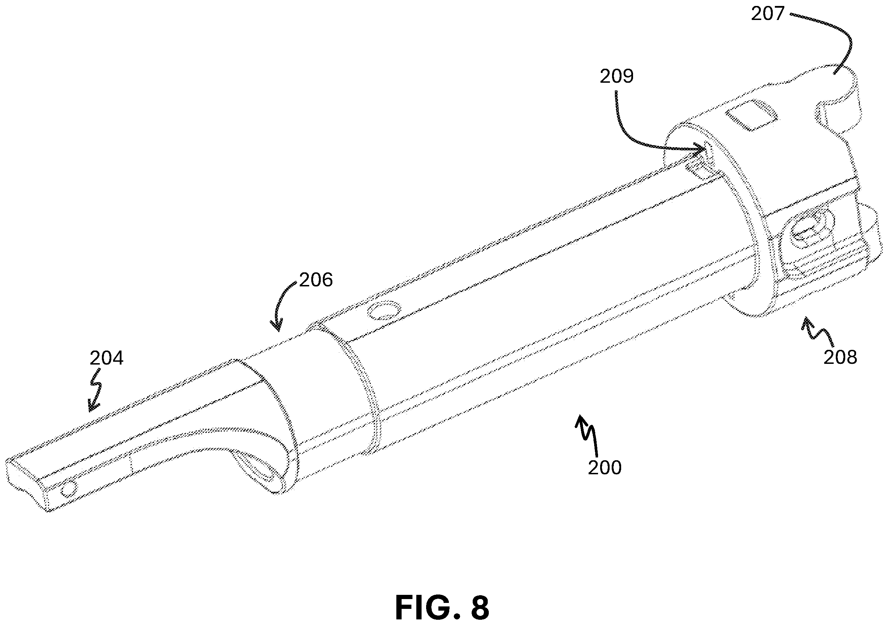

Shaft assembly (14) of the present example further includes a closure sleeve (90) which is slidably disposed along frame sleeve (200). Closure sleeve (90) is operable to transition between a distal position (FIG. 3A) along frame sleeve (200) and a proximal position (FIG. 3B) along frame sleeve (200), to thereby drive end effector (16) between an open configuration (FIG. 3A) and a closed configuration (FIG. 3B) as described in greater detail below. Such longitudinal movement of closure sleeve (90) is driven by a pair of closure beams (70), which are shown in FIGS. 4-7. Closure beams (70) are angularly offset from each other by 180° about the longitudinal axis (LA). In the present example, closure beams (70) are angularly offset from articulation bands (88) by 90° about the longitudinal axis (LA). Each closure beam (70) has a distal head (72) that is secured within a proximal portion (96) of closure sleeve (90), such that closure beams (70) are fixedly secured to closure sleeve (90) via heads (72). Closure beams (70) are slidably disposed in corresponding channels (209) in frame sleeve (200). Channels (209) are best seen in FIG. 8. Closure beams (70) may be coupled with corresponding actuation features within base assembly (12) to thereby drive coordinated longitudinal movement of closure beams (70). Such actuation features may be driven via corresponding input bodies (38) as described above.

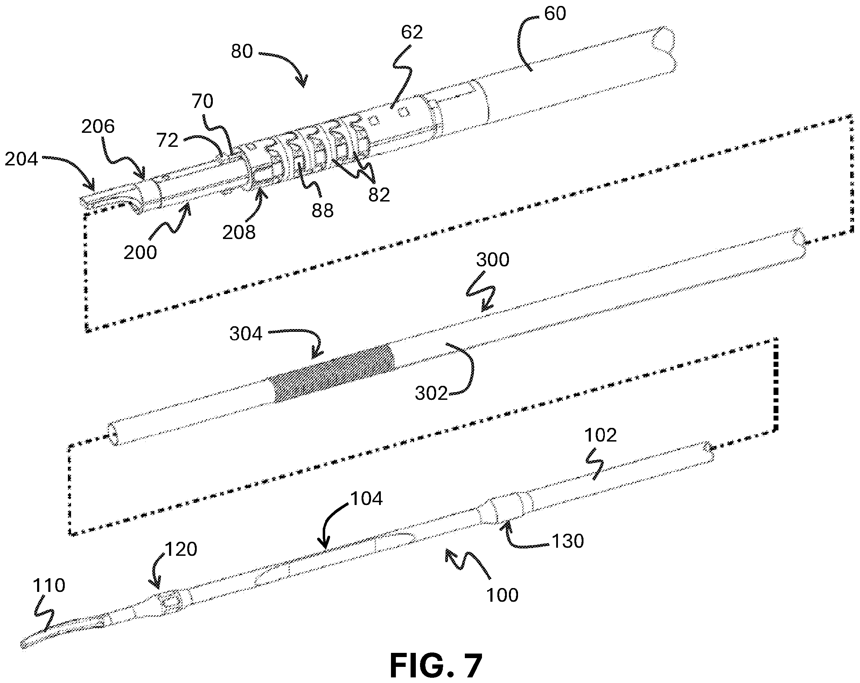

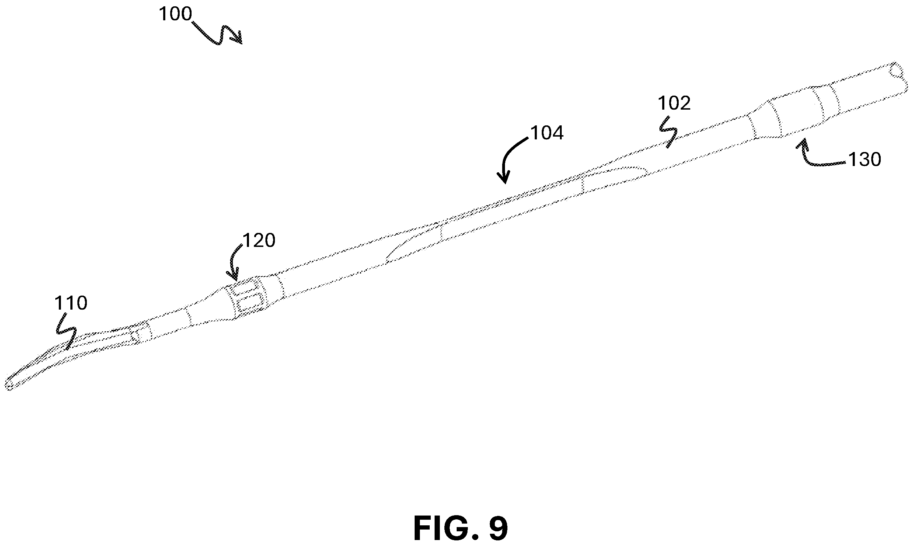

Shaft assembly (14) further includes an acoustic waveguide (100) and an elastomeric sleeve (300). As best seen in FIGS. 7 and 9, acoustic waveguide (100) includes a cylindraceous shaft (102), a distal blade (110), a distal nodal flange (120), and a proximal nodal flange (130). Each flange (120, 130) is enlarged relative to other portions of shaft (102), such that each flange (120, 130) has a larger diameter than other portions of shaft (102). A ribbon portion (104) is longitudinally interposed between flanges (120, 130). Ribbon portion (104) is substantially flat and planar, particularly as compared to cylindraceous shaft (102) in this example. Ribbon portion (104) is longitudinally positioned along articulation section (80) of shaft assembly (14), such that ribbon portion (104) is configured to flex to allow end effector (16) to be deflected to an articulated position. Distal blade (110) provides part of end effector (16) as will be described in greater detail below. Flanges (120, 130) are configured to provide structural support for acoustic waveguide (100) within shaft assembly (14) as will also be described in greater detail below. While only two flanges (120, 130) are shown, acoustic waveguide (100) may further include one or more additional flanges at nodal regions along shaft (102) at positions proximal to proximal nodal flange (130).

By being "nodal," flanges (120, 130) are located at positions along the length of acoustic waveguide (100) that correspond to nodes of vibrational waves when acoustic waveguide (100) vibrates at a resonant ultrasonic frequency. Thus, such nodes represent positions along the length of acoustic waveguide (100) that have minimal vibrational movement when acoustic waveguide (100) vibrates at a resonant ultrasonic frequency. The ultrasonic vibration of acoustic waveguide (100) may be driven by an ultrasonic transducer in base assembly (12), as described above. Thus, the proximal portion of acoustic waveguide (100) may be acoustically coupled with the ultrasonic transducer, with the ultrasonic vibrations being communicated along shaft assembly (14) to blade (110) via shaft (102).

The distal end of blade (110) is located at a position corresponding to an anti-node associated with resonant ultrasonic vibrations communicated through acoustic waveguide (100). In some versions, when the transducer assembly is energized, the distal end of blade (110) is configured to move longitudinally in the range of approximately 10 to 500 microns peak-to-peak, and in some instances in the range of about 20 to about 200 microns, at a predetermined vibratory frequency fₒ of, for example, 55.5 kHz. Alternatively, any other suitable range of displacement and/or frequency may be provided.

In some versions, acoustic waveguide (100) may be configured to amplify mechanical vibrations transmitted through acoustic waveguide (100). Furthermore, acoustic waveguide (100) may include features operable to control the gain of the vibrations along acoustic waveguide (100) and/or features to tune acoustic waveguide (100) to the resonant frequency of the acoustic system. Various suitable ways in which acoustic waveguide (100) may be mechanically and acoustically coupled with an ultrasonic transducer and/or otherwise configured will be apparent to those skilled in the art in view of the teachings herein.

Elastomeric sleeve (300) is radially interposed between acoustic waveguide (100) and other components of shaft assembly (14). Elastomeric sleeve (300) may comprise silicone, PEBAX, and/or any other suitable material(s). Elastomeric sleeve (300) of the present example includes a cylindrical body (302) with a corrugated portion (304). Corrugated portion (304) is longitudinally positioned along articulation section (80) of shaft assembly (14), such that corrugated portion (304) is configured to facilitate effective elongation of elastomeric sleeve (300) when articulation section (80) is bent to laterally deflect end effector (16) to be to an articulated position.

### C. Example of End Effector

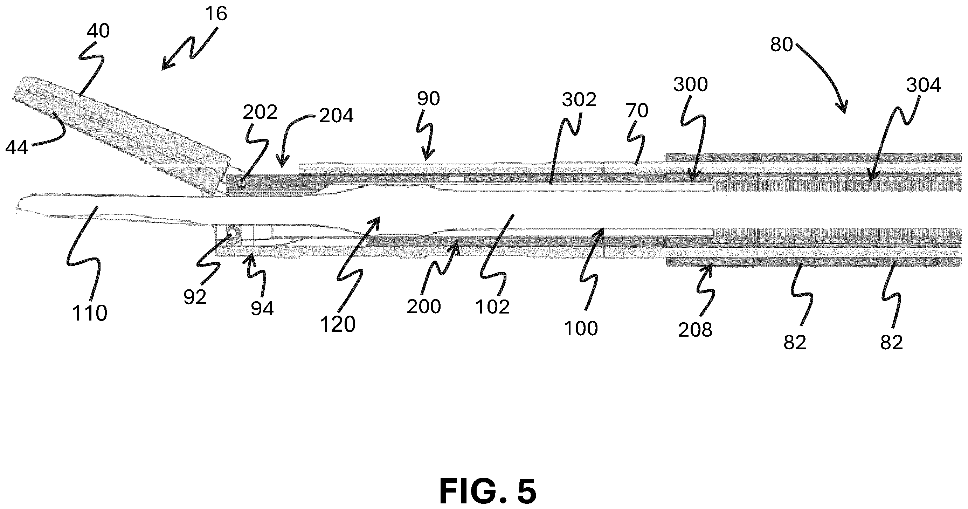

As best seen in FIGS. 3A-3B and 5, end effector (16) of the present example includes a clamp arm (40) and blade (110). A clamp pad (44) is secured to an underside of clamp arm (40) and faces blade (110). By way of example only, clamp pad (44) may comprise polytetrafluoroethylene (PTFE) and/or any other suitable material(s). A pair of ears (42) project downwardly from a proximal end of clamp arm (40). Clamp arm (40) is pivotally coupled with frame sleeve (200) and closure sleeve (90). Specifically, as best seen in FIG. 5, a first pivot pin (202) is disposed through a proximal end of clamp arm (40) and through a distally projecting tongue (204) of frame sleeve (200). A second pivot pin (92) is disposed through both ears (42) of clamp arm (40) and through a distally projecting tongue (94) of closure sleeve (90). As noted above, closure sleeve (90) may be translated longitudinally while frame sleeve (200) remains longitudinally stationary. Given the nature of the couplings via pins (92, 202), this longitudinal movement of closure sleeve (90) relative to frame sleeve (200) will transition end effector (16) between an open configuration (FIG. 3A) and a closed configuration (FIG. 3B).

By way of example only, when end effector (16) is in the open position (FIG. 3A), a tissue structure (e.g., vessel, etc.) may be positioned between clamp pad (44) and blade (110) while blade (110) is in a non-activated state (e.g., such that blade (110) is not ultrasonically vibrating). With the tissue structure suitably positioned between clamp pad (44) and blade (110), end effector (16) may be driven to the closed position (FIG. 3B) to thereby compress the tissue structure between clamp pad (44) and blade (110). In some cases, such operation may be utilized simply to grasp and stabilize, move, or otherwise manipulate the tissue structure, which may be subsequently released. In some other cases, blade (110) may be ultrasonically activated while the tissue structure is compressed between clamp pad (44) and blade (110). In some such cases, the combination of compression and ultrasonic vibration may seal the tissue structure (e.g., by denaturing proteins in the tissue). In addition, or in the alternative, the combination of compression and ultrasonic vibration may sever the tissue structure. Alternatively, end effector (16) may be used in any other suitable fashion.

In some versions, end effector (16) is rotatable relative to at least a portion of shaft assembly (14), about the longitudinal axis (LA), relative to base assembly (12). In some other versions, all or part of shaft assembly (14) is rotatable with end effector (16), about the longitudinal axis (LA), relative to base assembly (12). In either case, such rotation may be driven via actuation features within base assembly (12), which may in turn be driven via corresponding input bodies (38) as described above.

### II. Examples of Features to Ground Nodes of Acoustic Waveguide

As noted above, acoustic waveguide (100) has at least two nodal flanges, including distal nodal flange (120) and proximal nodal flange (130), with distal nodal flange (120) being positioned distally in relation to articulation section (80) and proximal nodal flange (130) being positioned proximally in relation to articulation section (80). It may be desirable to mechanically ground or secure distal nodal flange (120) relative to another portion of shaft assembly (14). In particular, it may be desirable to mechanically ground or secure distal nodal flange (120) relative to frame sleeve (200).

In some instances, mechanically grounding or securing distal nodal flange (120) relative to another portion of shaft assembly (14) (e.g., relative to frame sleeve (200)) may promote consistent alignment between distal blade (110) and clamp arm (40). This may in turn promote consistent clamping pressure profiles against tissue that is captured within end effector (16). In addition, or in the alternative, mechanically grounding or securing distal nodal flange (120) relative to another portion of shaft assembly (14) (e.g., relative to frame sleeve (200)) may minimize ultrasonic impedance in acoustic waveguide (100) and/or provide other results. The benefits of securely grounding distal nodal flange (120) relative to another portion of shaft assembly (14) may be particularly enhanced in ultrasonic surgical instruments that include articulation sections (e.g., like articulation section (80)), as bending of an acoustic waveguide at an articulation section may otherwise substantially compromise performance of the acoustic waveguide in cases where the distal portion of the acoustic waveguide is not sufficiently secured relative to a distal portion of the shaft assembly.

The following describes examples of features and arrangements that may be used to securely ground a distal nodal flange (e.g., distal nodal flange (120)) of an acoustic waveguide (e.g., acoustic waveguide (100)) relative to another portion of a shaft assembly (e.g., shaft assembly (14)). In some scenarios, these features and arrangements may provide a combination of axial and rotational constraints on relative movement between the acoustic waveguide and the other portion of the shaft assembly. In other words, these features and arrangements may substantially prevent or completely prevent axial and/or rotational relative movement between the acoustic waveguide and the other portion of the shaft assembly. Moreover, by securely grounding the distal nodal flange relative to another portion of the shaft assembly, the features and arrangements described below may provide a localized reaction force for bending loads encountered by the distal blade (e.g., distal blade (110)) when tissue is clamped against the distal blade (e.g., by clamp arm (40) and/or when other transversely oriented forces bear against the distal blade. Such a localized reaction force may in turn transfer the bending stress from the acoustic waveguide to the other portion of the shaft assembly to which the acoustic waveguide is securely grounded. This transfer of stress may in turn reduce stress and deflection of the portion of the acoustic waveguide that extends along the articulation section of the shaft assembly (e.g., ribbon portion (104)).

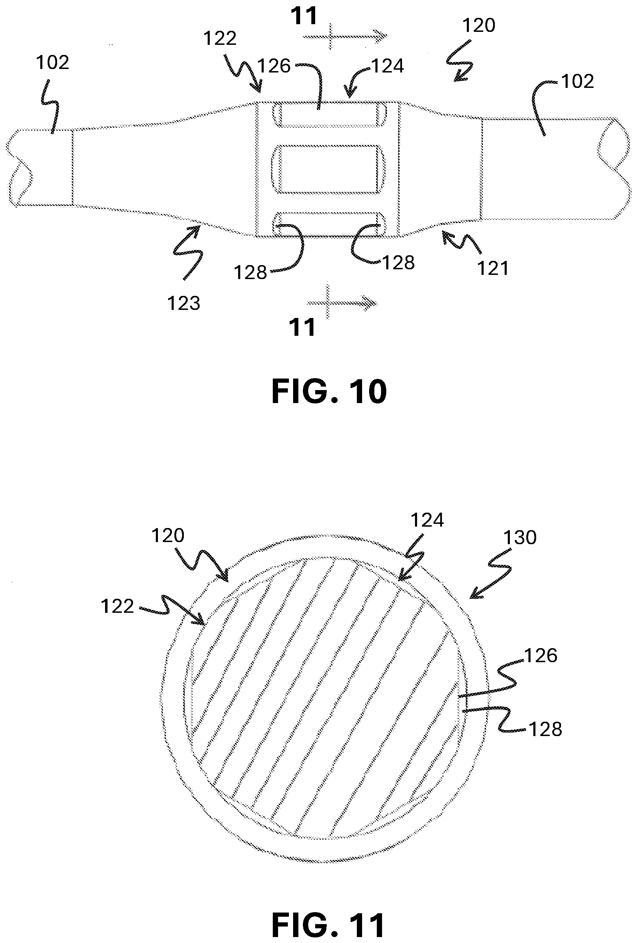

FIGS. 10-11 show distal nodal flange (120) in further detail. As shown, distal nodal flange (120) of this example includes an enlarged cylindraceous central region (122). A tapered proximal region (121) provides a proximal transition from shaft (102) to central region (122); while a tapered distal region (123) provides a distal transition from central region (122) to shaft (102). In some versions, proximal region (121) provides a single taper angle along a flat surface. In some other versions, proximal region (121) provides a compound taper with two or more taper angles along two or more flat surfaces. In still other versions, at least a portion of proximal region (121) has a concave curvature. Some such versions of proximal region (121) may provide a combination of concave curved surface(s) and flat taper surface(s). Similarly, some versions of distal region (123) provide a single taper angle along a flat surface. In some other versions, distal region (123) provides a compound taper with two or more taper angles along two or more flat surfaces. In still other versions, at least a portion of distal region (123) has a concave curvature. Some such versions of distal region (123) may provide a combination of concave curved surface(s) and flat taper surface(s). Alternatively, either region (121, 123) may have any other suitable configuration.

Distal nodal flange (120) of the present example further includes a set of recesses (124) formed along central region (122). Recesses (124) extend longitudinally across a substantial portion of the length of central region (122) (including the middle of the length of central region (122)); and are equidistantly angularly spaced apart from each other about the longitudinal axis (LA). A total of six recesses (124) are provided in this example, though any other suitable number of recesses (124) may be provided. With the number of recesses (124) being even, and with the angular spacing of recesses (124) about the longitudinal axis (LA) being equidistant, each recess (124) has a corresponding other recess (124) positioned 180° across from the recess (124). In other words, recesses (124) are aligned in diametrically opposed pairs. This alignment is best seen in FIG. 11.

As also best seen in FIG. 11, each recess (124) includes a flat recessed surface (126), though some other versions may include a recessed surface (126) having any other suitable profile (e.g., concave curved, roughened, etc.). Each recess (124) also includes a curved transition surface (128) at each longitudinal end of flat recessed surface (126) in the present example, though other configurations may be used. By way of example only, curved transition surface (128) may instead be flat but angled, etc.

In the present example, distal nodal flange (120) is mechanically grounded or secured to frame sleeve (200) by crimping a crimping region (206) of frame sleeve (200) around distal nodal flange (120). This crimping is done while a distal portion of cylindrical body (302) of elastomeric sleeve (300) is radially interposed between frame sleeve (200) and distal nodal flange (120), such that cylindrical body (302) is compressed between crimping region (206) and distal nodal flange (120) when crimping region (206) is crimped around distal nodal flange (120).

The crimping of crimping region (206) around distal nodal flange (120) may be carried out in numerous ways. By way of example only, crimping region (206) and distal nodal flange (120) may be positioned within a chuck or other clamping device that applies forces that are directed radially inwardly, simultaneously along several different radii extending from the longitudinal axis (LA). In some other versions, a clamping member may apply clamping forces along two diametrically opposed radii simultaneously, be repositioned at a different angular position, then again apply clamping forces along two other diametrically opposed radii simultaneously; and this process may be repeated until the diametrically opposed clamping forces are applied at the desired number of angular positions. Alternatively, the crimping of crimping region (206) around distal nodal flange (120) may be carried out in any other suitable fashion using any other suitable techniques and/or equipment.

Frame sleeve (200) of the present example comprises a malleable material (e.g., steel, titanium, polyether ether ketone, etc.). Thus, when crimping region (206) is crimped around distal nodal flange (120), the material of crimping region (206) deforms inwardly, firmly pressing cylindrical body (302) of elastomeric sleeve (300) around distal nodal flange (120); and maintains the deformed configuration. Thus, the deformed crimping region (206) provides a firm grip on distal nodal flange (120) to thereby mechanically ground or secure distal nodal flange (120) relative to frame sleeve (200).

In the present example, deformed portions of cylindrical body (302) of elastomeric sleeve (300) are received in recesses (124) when crimping region (206) is crimped around distal nodal flange (120), such that portions of cylindrical body (302) of elastomeric sleeve (300) contact surfaces (126, 128) of recess (124) when crimping region (206) is crimped around distal nodal flange (120). In some versions, at least a portion of crimping region (206) is also received in recesses (124) when crimping region (206) is crimped around distal nodal flange (120). The receipt of cylindrical body (302) (and, in some cases, at least a portion of crimping region (206)) in recesses (124) may provide a firm gripping relationship between distal nodal flange (120) and frame sleeve (200). In other words, the presence of recesses (124) may enhance the grip strength relative to the grip strength that might otherwise be provided in versions of distal nodal flange (120) that lack recesses (124). It should also be understood that flat recessed surfaces (126) may provide enhanced securement against rotation of acoustic waveguide (100) relative to frame sleeve (200) about the longitudinal axis (LA); while curved transition surfaces (128) may provide enhanced securement against translation of acoustic waveguide (100) relative to frame sleeve (200) along the longitudinal axis (LA).

In some cases, the presence of elastomeric sleeve (300) between frame sleeve (200) and distal nodal flange (120) may provide a damper that minimizes transfer of vibration from acoustic waveguide (100) to frame sleeve (200). In addition, or in the alternative, the presence of elastomeric sleeve (300) between frame sleeve (200) and distal nodal flange (120) may enhance the grip between crimping region (206) and distal nodal flange (120). In addition, or in the alternative, the presence of elastomeric sleeve (300) between frame sleeve (200) and distal nodal flange (120) may provide an enhanced fluid tight seal between the inner surface of frame sleeve (200) and distal nodal flange (120).

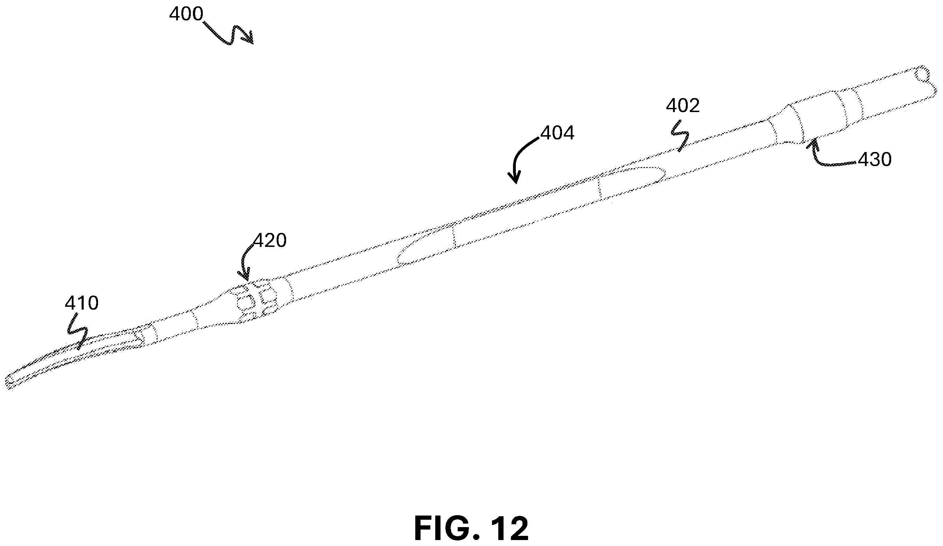

FIG. 12 shows an example of another acoustic waveguide (400) that may be incorporated into ultrasonic surgical instrument (10) in place of acoustic waveguide (100). Acoustic waveguide (400) of this example may be configured and operable just like acoustic waveguide (100), except for the differences described below. Acoustic waveguide (400) of this example includes a cylindraceous shaft (402), a distal blade (410), a distal nodal flange (420), and a proximal nodal flange (430). Each flange (420, 430) is enlarged relative to other portions of shaft (402), such that each flange (420, 430) has a larger diameter than other portions of shaft (402). A ribbon portion (404) is longitudinally interposed between flanges (420, 430). Ribbon portion (404) is substantially flat and planar, particularly as compared to cylindraceous shaft (402) in this example. Ribbon portion (404) is configured to be longitudinally positioned along articulation section (80) of shaft assembly (14), such that ribbon portion (404) is configured to flex to allow end effector (16) to be deflected to an articulated position. Distal blade (410) is configured to provide part of end effector (16) as described above. Flanges (420, 430) are configured to provide structural support for acoustic waveguide (400) within shaft assembly (14) as also described above. While only two flanges (420, 430) are shown, acoustic waveguide (400) may further include one or more additional flanges at nodal regions along shaft (402) at positions proximal to proximal nodal flange (430).

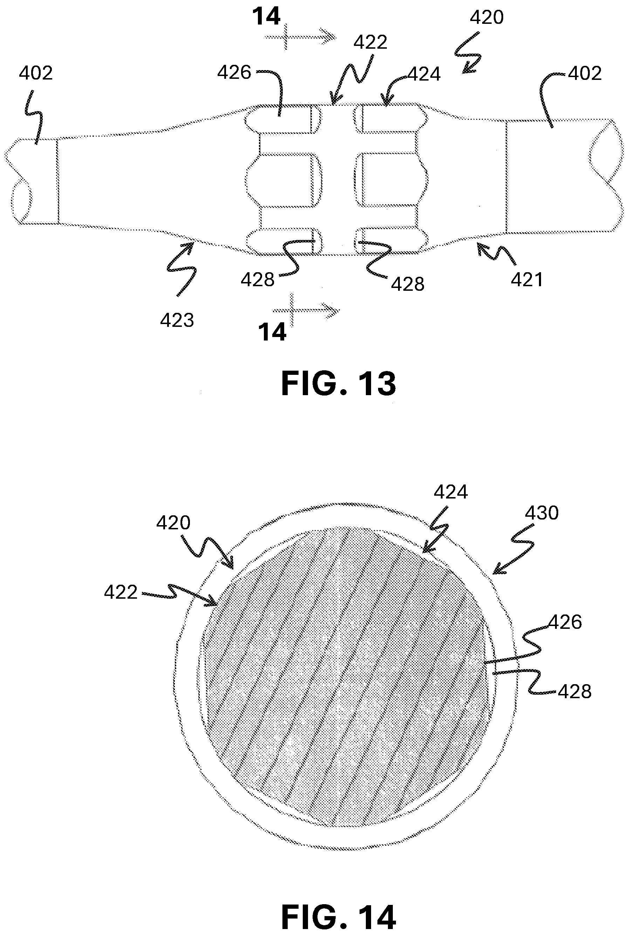

FIGS. 13-14 show distal nodal flange (420) in further detail. As shown, distal nodal flange (420) of this example includes an enlarged cylindraceous central region (422). A tapered proximal region (421) provides a proximal transition from shaft (402) to central region (422); while a tapered distal region (423) provides a distal transition from central region (422) to shaft (402). In some versions, proximal region (421) provides a single taper angle along a flat surface. In some other versions, proximal region (421) provides a compound taper with two or more taper angles along two or more flat surfaces. In still other versions, at least a portion of proximal region (421) has a concave curvature. Some such versions of proximal region (421) may provide a combination of concave curved surface(s) and flat taper surface(s). Similarly, some versions of distal region (423) provide a single taper angle along a flat surface. In some other versions, distal region (423) provides a compound taper with two or more taper angles along two or more flat surfaces. In still other versions, at least a portion of distal region (423) has a concave curvature. Some such versions of distal region (423) may provide a combination of concave curved surface(s) and flat taper surface(s). Alternatively, either region (421, 423) may have any other suitable configuration.

Distal nodal flange (420) of the present example further includes a set of recesses (424) formed along central region (422). A proximal set of recesses (424) extend longitudinally along a proximal portion of central region (422); while a distal set of recesses (424) extend longitudinally along a distal portion of central region (422). A longitudinally intermediate portion of central region (422) thus provides a structural interruption between the proximal set of recesses (424) and the distal set of recesses (424). In some variations, the diameter of the longitudinally intermediate portion of central region (422) is greater than the diameter of the rest of the longitudinally intermediate portion of central region (422).

All recesses (424) of the proximal set of recesses (424) are equidistantly angularly spaced apart from each other about the longitudinal axis (LA). Likewise, all recesses (424) of the distal set of recesses (424) are equidistantly angularly spaced apart from each other about the longitudinal axis (LA). A total of six distal recesses (424) and six proximal recesses (424) are provided in this example, though any other suitable number of recesses (424) may be provided. With the number of recesses (424) in each set being even, and with the angular spacing of each set of recesses (424) about the longitudinal axis (LA) being equidistant, each recess (424) of each set has a corresponding other recess (424) in the same set positioned 180° across from the recess (424). In other words, recesses (424) of each set are aligned in diametrically opposed pairs. This alignment is best seen in FIG. 14. While there are an even number of recesses (424) in this example, other versions may have an odd number of recesses (424).

As also best seen in FIG. 14, each recess (424) includes a flat recessed surface (426), though some other versions may include a recessed surface (426) having any other suitable profile (e.g., concave curved, roughened, etc.). Each recess (424) also includes a curved transition surface (428) at one longitudinal end of flat recessed surface (426) in the present example, though other configurations may be used. By way of example only, curved transition surface (428) may instead be flat but angled, etc.

In the present example, distal nodal flange (420) may be mechanically grounded or secured to frame sleeve (200) by crimping a crimping region (206) of frame sleeve (200) around distal nodal flange (420). This crimping may be done while a distal portion of cylindrical body (302) of elastomeric sleeve (300) is radially interposed between frame sleeve (200) and distal nodal flange (420), such that cylindrical body (302) is compressed between crimping region (206) and distal nodal flange (420) when crimping region (206) is crimped around distal nodal flange (420). This crimping may be carried out in accordance with the teachings provided above in the context of crimping the crimping region (206) of frame sleeve (200) around distal nodal flange (120). As described above, the deformed crimping region (206) may provide a firm grip on distal nodal flange (420) to thereby mechanically ground or secure distal nodal flange (420) relative to frame sleeve (200). As also described above, the presence of recesses (424) may enhance the grip strength relative to the grip strength that might otherwise be provided in versions of distal nodal flange (420) that lack recesses (424). It should also be understood that flat recessed surfaces (426) may provide enhanced securement against rotation of acoustic waveguide (400) relative to frame sleeve (200) about the longitudinal axis (LA); while curved transition surfaces (428) may provide enhanced securement against translation of acoustic waveguide (400) relative to frame sleeve (200) along the longitudinal axis (LA).

In some cases, the presence of elastomeric sleeve (300) between frame sleeve (200) and distal nodal flange (420) may provide a damper that minimizes transfer of vibration from acoustic waveguide (400) to frame sleeve (200). In addition, or in the alternative, the presence of elastomeric sleeve (300) between frame sleeve (200) and distal nodal flange (420) may enhance the grip between crimping region (206) and distal nodal flange (420). In addition, or in the alternative, the presence of elastomeric sleeve (300) between frame sleeve (200) and distal nodal flange (420) may provide an enhanced fluid tight seal between the inner surface of frame sleeve (200) and distal nodal flange (420).

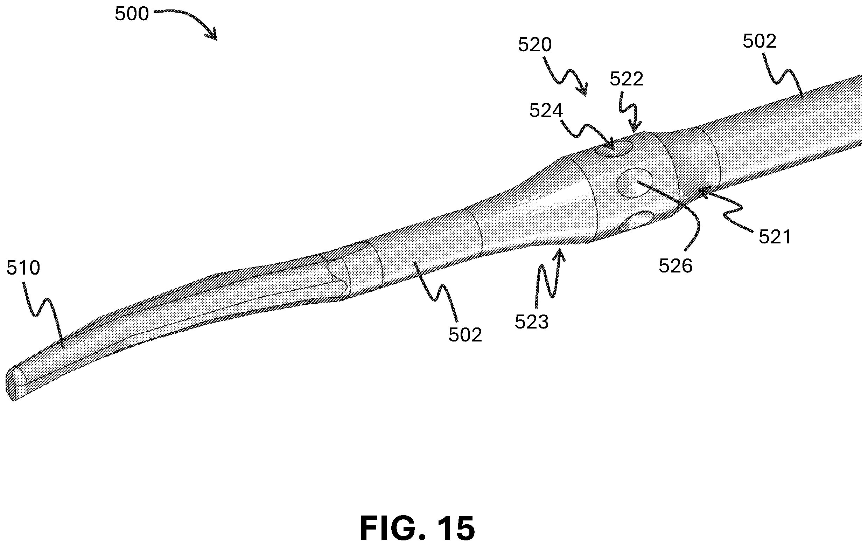

FIG. 15 shows an example of another acoustic waveguide (500) that may be incorporated into ultrasonic surgical instrument (10) in place of acoustic waveguide (100). Acoustic waveguide (500) of this example may be configured and operable just like acoustic waveguide (100), except for the differences described below. Acoustic waveguide (500) of this example includes a cylindraceous shaft (402), a distal blade (510), and a distal nodal flange (520). Distal nodal flange (520) is enlarged relative to other portions of shaft (502), such that distal nodal flange (520) has a larger diameter than other portions of shaft (502). Acoustic waveguide (500) may also include a proximal nodal flange (not shown), a ribbon portion (not shown), other nodal flanges (not shown), and/or other structural features. Distal blade (510) is configured to provide part of end effector (16) as described above. Distal nodal flange (520) is configured to provide structural support for acoustic waveguide (500) within shaft assembly (14) as also described above.

As shown, distal nodal flange (520) of this example includes an enlarged cylindraceous central region (522). A tapered proximal region (521) provides a proximal transition from shaft (502) to central region (522); while a tapered distal region (523) provides a distal transition from central region (522) to shaft (502). In some versions, proximal region (521) provides a single taper angle along a flat surface. In some other versions, proximal region (521) provides a compound taper with two or more taper angles along two or more flat surfaces. In still other versions, at least a portion of proximal region (521) has a concave curvature. Some such versions of proximal region (521) may provide a combination of concave curved surface(s) and flat taper surface(s). Similarly, some versions of distal region (523) provide a single taper angle along a flat surface. In some other versions, distal region (523) provides a compound taper with two or more taper angles along two or more flat surfaces. In still other versions, at least a portion of distal region (523) has a concave curvature. Some such versions of distal region (523) may provide a combination of concave curved surface(s) and flat taper surface(s). Alternatively, either region (521, 523) may have any other suitable configuration.

Distal nodal flange (520) of the present example further includes a set of recesses (524) formed along central region (522). Recesses (524) are longitudinally centered along the length of central region (522) in this example. Recesses (524) are equidistantly angularly spaced apart from each other about the longitudinal axis (LA). A total of six recesses (524) are provided in this example, though any other suitable number of recesses (524) may be provided. With the number of recesses (524) being even, and with the angular spacing of recesses (524) about the longitudinal axis (LA) being equidistant, each recess (524) has a corresponding other recess (524) positioned 180° across from the recess (524). In other words, recesses (524) are aligned in diametrically opposed pairs. While there are an even number of recesses (524) in this example, other versions may have an odd number of recesses (524).

Each recess (524) of the present example includes a concave recessed surface (526). In some versions, each recessed surface (526) is curved, with the curve being defined by one or more radii. In some other versions, each recessed surface (526) has a negative conical shape. Alternatively, each recessed surface (526) may have any other suitable configuration.

In the present example, distal nodal flange (520) may be mechanically grounded or secured to frame sleeve (200) by crimping a crimping region (206) of frame sleeve (200) around distal nodal flange (520). This crimping may be done while a distal portion of cylindrical body (302) of elastomeric sleeve (300) is radially interposed between frame sleeve (200) and distal nodal flange (520), such that cylindrical body (302) is compressed between crimping region (206) and distal nodal flange (520) when crimping region (206) is crimped around distal nodal flange (520). This crimping may be carried out in accordance with the teachings provided above in the context of crimping the crimping region (206) of frame sleeve (200) around distal nodal flange (120). As described above, the deformed crimping region (206) may provide a firm grip on distal nodal flange (520) to thereby mechanically ground or secure distal nodal flange (520) relative to frame sleeve (200). As also described above, the presence of recesses (524) may enhance the grip strength relative to the grip strength that might otherwise be provided in versions of distal nodal flange (520) that lack recesses (524). It should also be understood that recessed surfaces (526) may provide enhanced securement against both rotation of acoustic waveguide (500) relative to frame sleeve (200) about the longitudinal axis (LA); and translation of acoustic waveguide (500) relative to frame sleeve (200) along the longitudinal axis (LA).

In some cases, the presence of elastomeric sleeve (300) between frame sleeve (200) and distal nodal flange (520) may provide a damper that minimizes transfer of vibration from acoustic waveguide (500) to frame sleeve (200). In addition, or in the alternative, the presence of elastomeric sleeve (300) between frame sleeve (200) and distal nodal flange (520) may enhance the grip between crimping region (206) and distal nodal flange (520). In addition, or in the alternative, the presence of elastomeric sleeve (300) between frame sleeve (200) and distal nodal flange (520) may provide an enhanced fluid tight seal between the inner surface of frame sleeve (200) and distal nodal flange (520).

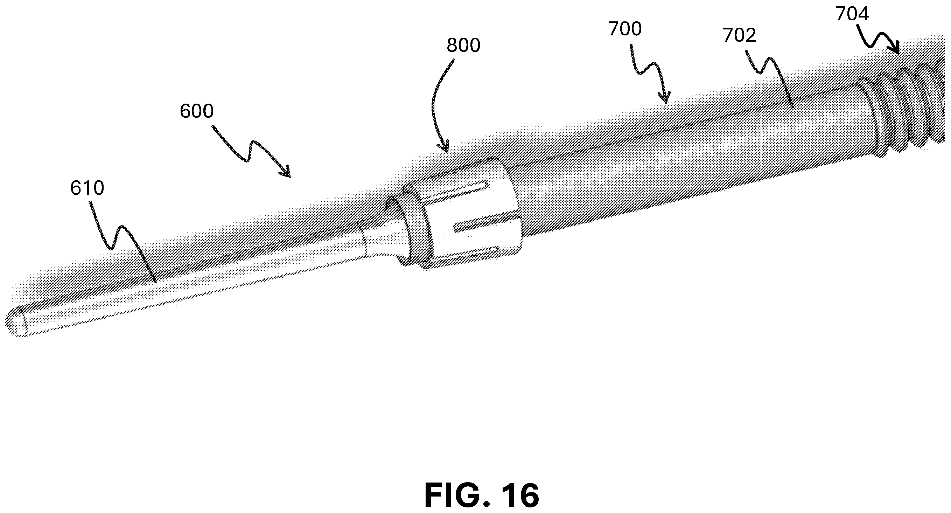

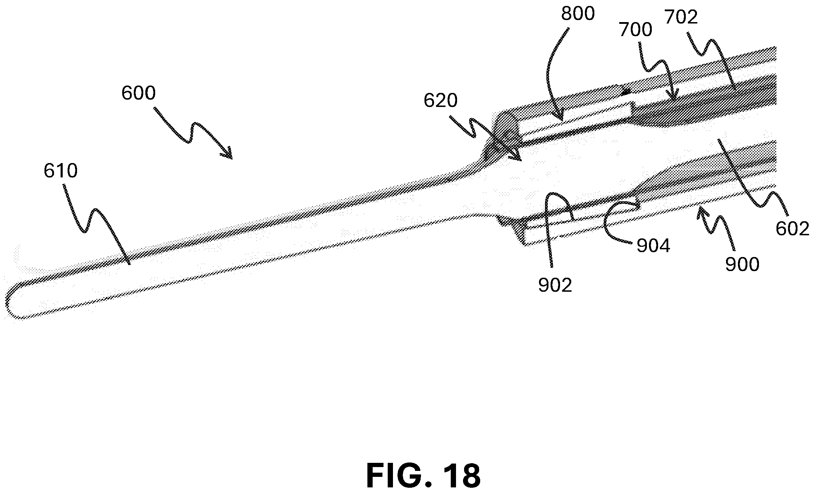

FIGS. 16 and 18 show an example of another acoustic waveguide (600) that may be incorporated into ultrasonic surgical instrument (10) in place of acoustic waveguide (100). Acoustic waveguide (600) of this example may be configured and operable just like acoustic waveguide (100), except for the differences described below. Acoustic waveguide (600) of this example includes a cylindraceous shaft (602), a distal blade (610), and a distal nodal flange (620). Acoustic waveguide (600) may also include a proximal nodal flange (not shown), a ribbon portion (not shown), other nodal flanges (not shown), and/or other structural features. Distal blade (610) is configured to provide part of end effector (16) as described above. Distal nodal flange (620) is configured to provide structural support for acoustic waveguide (600) within shaft assembly (14) as also described above. Distal nodal flange (620) is enlarged relative to other portions of shaft (602), such that distal nodal flange (620) has a larger diameter than other portions of shaft (602). While not shown in FIGS. 16 or 18, distal nodal flange (620) may include recesses like any of the recesses (124, 424, 524) described herein; or may have any other suitable structural features.

As also shown in FIGS. 16 and 18, an elastomeric sleeve (700), a collet (800), and an outer sleeve (900) may also be combined with acoustic waveguide (600) to form part of a variation of shaft assembly (14). Elastomeric sleeve (700) may comprise silicone, PEBAX, and/or any other suitable material(s). Elastomeric sleeve (700) of this example includes a cylindrical body (702) with a corrugated portion (704), such that elastomeric sleeve (700) of this example is configured and operable like elastomeric sleeve (300) described above. Collet (800) is configured to crimp against distal nodal flange (620) like crimping region (206) of frame sleeve (200) described above. Collet (800) may comprise a malleable material (e.g., steel, titanium, polyether ether ketone, etc.).

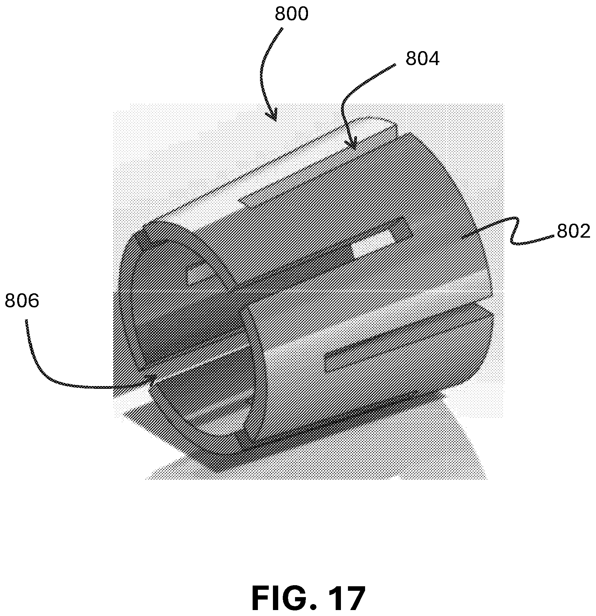

As best seen in FIG. 17, collet (800) includes a hollow frustoconical shaped body (802) with a set of distal proximal slits (804) and a set of distal slits (806). Proximal slits (804) extend longitudinally from the proximal edge of body (802), stopping short of the distal edge of body (802). Proximal slits (804) are angularly spaced equidistantly apart from each other. Distal slits (806) extend longitudinally from the distal edge of body (802), stopping short of the proximal edge of body (802). Distal slits (806) are angularly spaced equidistantly apart from each other. Distal slits (806) are angularly offset relative to proximal slits (804) such that slits (804, 806) form an interdigitated arrangement. In the present example, slits (804, 806) are configured to promote uniform deformation of collet (800) around distal nodal flange (620) during crimping of collet (800) around distal nodal flange (620).

As shown in FIG. 18, outer sleeve (900) includes a tapered inner distal surface (902) that proximally terminates in a shelf (904). During an assembly process, tapered inner distal surface (902) is configured to bear radially inwardly against collet (800) to thereby crimp/deform collet (800) radially inwardly against distal nodal flange (620), with cylindrical body (702) of elastomeric sleeve (700) being captured/compressed between collet (800) and distal nodal flange (620). The proximal edge of body (802) engages shelf (904). This relative positioning is subsequently maintained to form a distal portion of the shaft assembly. In some variations, collet (800) is merely flexible yet not necessarily malleable. In such variations, outer sleeve (900) may still compress collet (800) radially inwardly against distal nodal flange (620) and maintain such compression. Regardless of whether collet (800) is malleable or merely flexible, when collet (800) is in the configuration shown in FIG. 18, distal nodal flange (620) may be mechanically grounded or secured relative to outer sleeve (900).

In some cases, the presence of elastomeric sleeve (700) between collet (800) and distal nodal flange (620) may provide a damper that minimizes transfer of vibration from acoustic waveguide (600) to collet (800). In addition, or in the alternative, the presence of elastomeric sleeve (300) between collet (800) and distal nodal flange (620) may enhance the grip between collet (800) and distal nodal flange (620). In addition, or in the alternative, the presence of elastomeric sleeve (300) between collet (800) and distal nodal flange (620) may provide an enhanced fluid tight seal between the inner surface of collet (800) and distal nodal flange (620).

### IV. Miscellaneous

Versions of the devices described above may have application in conventional medical treatments and procedures conducted by a medical professional, as well as application in robotic-assisted medical treatments and procedures. By way of example only, the various teachings herein may be readily combined with various teachings of any of the references cited herein relating to robotic surgical systems.

Versions described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by an operator immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An ultrasonic surgical instrument (10), comprising:
(a) a shaft assembly (14) comprising:
(i) an acoustic waveguide (100, 400, 500, 600), the acoustic waveguide being configured to transmit ultrasonic vibrations, the acoustic waveguide having a distal nodal flange (120, 420, 520, 620), and
(ii) a distal portion (206, 800, 902) bearing radially inwardly against the distal nodal flange, the distal portion cooperating with the distal nodal flange to prevent rotational movement of the distal nodal flange relative to the distal portion and to prevent longitudinal movement of the distal nodal flange relative to the distal portion;
(b) an end effector (16) at a distal end of the shaft assembly, the end effector including:
(i) an ultrasonic blade (110, 410, 510, 610), the ultrasonic blade being positioned at a distal end of the acoustic waveguide, and
(ii) a clamp arm (40) operable to pivot toward and away from the ultrasonic blade,
wherein the distal portion includes a frame sleeve (200, 800) positioned coaxially about the distal nodal flange, the frame sleeve having a crimping section (206, 800), the crimping section being crimped about the distal nodal flange to thereby bear radially inwardly against the distal nodal flange.

2. The ultrasonic surgical instrument of claim 1, the distal nodal flange including a plurality of recesses (124, 424, 524).

3. The ultrasonic surgical instrument of claim 2, the recesses extending longitudinally across a middle of a central region (122, 522) along a length of the distal nodal flange.

4. The ultrasonic surgical instrument of claim 2, the recesses including a distal set of recesses (424) and a proximal set of recesses (424), the distal set of recesses being positioned distally in relation to a longitudinally intermediate portion of a central region (422) along a length of the distal nodal flange, the proximal set of recesses being positioned proximally in relation to the longitudinally intermediate portion of the central region, the longitudinally intermediate portion of the central region providing a structural interruption between the distal set of recesses and the proximal set of recesses.

5. The ultrasonic surgical instrument of any one of claims 2 to 4, each recess of the plurality of recesses being defined at least in part by a first recessed surface (126, 426) and a second recessed surface (128, 428), the second recessed surface providing a transition to the first recessed surface from other portions of the distal nodal flange.

6. The ultrasonic surgical instrument of claim 5, the first recessed surface being flat.

7. The ultrasonic surgical instrument of claim 5 or claim 6, the second recessed surface being curved.

8. The ultrasonic surgical instrument of any one of claims 2 to 7, each recess having a concave shape, optionally each recess having a having a curved surface defining the concave shape, and/or each recess having a negative conical surface defining the concave shape.

9. The ultrasonic surgical instrument of any preceding claim, the crimping section being malleable.

10. The ultrasonic surgical instrument of any preceding claim, the clamp arm being pivotally coupled with the frame sleeve.

11. The ultrasonic surgical instrument of any preceding claim, the shaft assembly further including an articulation section (80), the articulation section being operable to deflect the end effector laterally away from a central longitudinal axis (LA) of the shaft assembly.

12. The ultrasonic surgical instrument of claim 11, the acoustic waveguide further comprising a flexible region extending along the articulation section, optionally the distal nodal flange being positioned distal to the flexible region.

13. The ultrasonic surgical instrument of any preceding claim, the shaft assembly further comprising an elastomeric member (300, 700) interposed between the distal nodal flange and the distal portion of the shaft assembly, the elastomeric member being compressed against the distal nodal flange by the distal portion of the shaft assembly.

14. The ultrasonic surgical instrument of claim 1, wherein the shaft assembly further comprises an articulation section (80), the distal nodal flange being positioned distally in relation to the articulation section, the articulation section being operable to deflect the end effector laterally away from a central longitudinal axis of the shaft assembly.

15. A method, comprising:
(a) positioning an elastomeric feature (300,700) about a distal nodal flange (120, 420, 520, 620) of an acoustic waveguide (100, 400, 500, 600) of a shaft assembly (14) of an ultrasonic surgical instrument (10);
(b) positioning a malleable sleeve (200, 800) about the distal nodal flange such that the elastomeric feature is radially interposed between the malleable sleeve and the distal nodal flange; and
(c) compressing the malleable sleeve radially inwardly to thereby crimp the malleable sleeve against the distal nodal flange and to thereby compress the elastomeric feature against the distal nodal flange, the malleable properties of the sleeve being configured to maintain the crimp and compression, thereby fixedly securing the malleable sleeve relative to the distal nodal flange.

## Patentansprüche

1. Chirurgisches Ultraschallinstrument (10), umfassend:
(a) eine Schaftanordnung (14), umfassend:
(i) einen akustischen Wellenleiter (100, 400, 500, 600), wobei der akustische Wellenleiter konfiguriert ist, um Ultraschallschwingungen zu übertragen, wobei der akustische Wellenleiter einen distalen Knotenflansch (120, 420, 520, 620) aufweist, und
(ii) einen distalen Abschnitt (206, 800, 902), der radial nach innen gegen den distalen Knotenflansch anliegt, wobei der distale Abschnitt mit dem distalen Knotenflansch zusammenwirkt, um eine Rotationsbewegung des distalen Knotenflanschs relativ zu dem distalen Abschnitt zu verhindern und um eine Längsbewegung des distalen Knotenflanschs relativ zu dem distalen Abschnitt zu verhindern;
(b) einen Endeffektor (16) an einem distalen Ende der Schaftanordnung, wobei der Endeffektor einschließt:
(i) eine Ultraschallklinge (110, 410, 510, 610), wobei die Ultraschallklinge an einem distalen Ende des akustischen Wellenleiters positioniert ist, und
(ii) einen Klemmarm (40), der betriebsfähig ist, um zu der Ultraschallklinge hin und von dieser weg zu schwenken,
wobei der distale Abschnitt eine Rahmenhülse (200, 800) einschließt, die um den distalen Knotenflansch koaxial positioniert ist, wobei die Rahmenhülse einen Crimpbereich (206, 800) aufweist, wobei der Crimpbereich um den distalen Knotenflansch gecrimpt ist, um dadurch radial nach innen gegen den distalen Knotenflansch anzuliegen.

2. Chirurgisches Ultraschallinstrument nach Anspruch 1, wobei der distale Knotenflansch eine Vielzahl von Vertiefungen (124, 424, 524) einschließt.

3. Chirurgisches Ultraschallinstrument nach Anspruch 2, wobei sich die Vertiefungen längs über eine Mitte einer zentralen Region (122, 522) entlang einer Länge des distalen Knotenflanschs erstrecken.

4. Chirurgisches Ultraschallinstrument nach Anspruch 2, wobei die Vertiefungen einen distalen Satz von Vertiefungen (424) und einen proximalen Satz von Vertiefungen (424) einschließen, wobei der distale Satz von Vertiefungen distal in Bezug auf einen längs-zwischenliegenden Abschnitt einer zentralen Region (422) entlang einer Länge des distalen Knotenflanschs positioniert ist, wobei der proximale Satz von Vertiefungen proximal in Bezug auf den längs-zwischenliegenden Abschnitt der zentralen Region positioniert ist, wobei der längs-zwischenliegende Abschnitt der zentralen Region eine strukturelle Unterbrechung zwischen dem distalen Satz von Vertiefungen und dem proximalen Satz von Vertiefungen bereitstellt.

5. Chirurgisches Ultraschallinstrument nach einem der Ansprüche 2 bis 4, wobei jede Vertiefung der Vielzahl von Vertiefungen mindestens teilweise durch eine erste vertiefte Oberfläche (126, 426) und eine zweite vertiefte Oberfläche (128, 428) definiert ist, wobei die zweite vertiefte Oberfläche einen Übergang zu der ersten vertieften Oberfläche von anderen Abschnitten des distalen Knotenflanschs bereitstellt.

6. Chirurgisches Ultraschallinstrument nach Anspruch 5, wobei die erste vertiefte Oberfläche flach ist.

7. Chirurgisches Ultraschallinstrument nach Anspruch 5 oder 6, wobei die zweite vertiefte Oberfläche gekrümmt ist.

8. Chirurgisches Ultraschallinstrument nach einem der Ansprüche 2 bis 7, wobei jede Vertiefung eine konkave Form aufweist, optional jede Vertiefung eine gekrümmte Oberfläche aufweist, die die konkave Form definiert, und/oder jede Vertiefung eine negative konische Oberfläche aufweist, die die konkave Form definiert.

9. Chirurgisches Ultraschallinstrument nach einem der vorstehenden Ansprüche, wobei der Crimpbereich verformbar ist.

10. Chirurgisches Ultraschallinstrument nach einem der vorstehenden Ansprüche, wobei der Klemmarm schwenkbar mit der Rahmenhülse gekoppelt ist.

11. Chirurgisches Ultraschallinstrument nach einem der vorstehenden Ansprüche, wobei die Schaftanordnung ferner einen Gelenkbereich (80) einschließt, wobei der Gelenkbereich betriebsfähig ist, um den Endeffektor lateral von einer zentralen Längsachse (LA) der Schaftanordnung weg abzulenken.

12. Chirurgisches Ultraschallinstrument nach Anspruch 11, der akustische Wellenleiter ferner umfassend eine flexible Region, die sich entlang des Gelenkbereichs erstreckt, wobei optional der distale Knotenflansch distal zu der flexiblen Region positioniert ist.

13. Chirurgisches Ultraschallinstrument nach einem der vorstehenden Ansprüche, die Schaftanordnung ferner umfassend ein elastomeres Element (300, 700), das zwischen dem distalen Knotenflansch und dem distalen Abschnitt der Schaftanordnung eingerichtet ist, wobei das elastomere Element durch den distalen Abschnitt der Schaftanordnung gegen den distalen Knotenflansch gedrückt wird.

14. Chirurgisches Ultraschallinstrument nach Anspruch 1, wobei die Schaftanordnung ferner einen Gelenkbereich (80) umfasst, wobei der distale Knotenflansch distal in Bezug auf den Gelenkbereich positioniert ist, wobei der Gelenkbereich betriebsfähig ist, um den Endeffektor lateral von einer zentralen Längsachse der Schaftanordnung weg abzulenken.

15. Verfahren, umfassend:
(a) Positionieren eines elastomeren Merkmals (300, 700) um einen distalen Knotenflansch (120, 420, 520, 620) eines akustischen Wellenleiters (100, 400, 500, 600) einer Schaftanordnung (14) eines chirurgischen Ultraschallinstruments (10) herum;
(b) derartiges Positionieren einer verformbaren Hülse (200, 800) um den distalen Knotenflansch herum, dass das elastomere Merkmal radial zwischen der verformbaren Hülse und dem distalen Knotenflansch eingerichtet ist; und
(c) radiales Zusammendrücken der formbaren Hülse nach innen, um dadurch die verformbare Hülse gegen den distalen Knotenflansch zu crimpen und um dadurch das elastomere Merkmal gegen den distalen Knotenflansch zu komprimieren, wobei die verformbaren Eigenschaften der Hülse konfiguriert sind, um das Crimpen und die Kompression aufrechtzuerhalten, wobei dadurch die verformbare Hülse relativ zu dem distalen Knotenflansch fest gesichert wird.

## Revendications

1. Instrument chirurgical à ultrasons (10), comprenant :
(a) un ensemble tige (14) comprenant :
(i) un guide d'ondes acoustique (100, 400, 500, 600), le guide d'ondes acoustique étant configuré pour transmettre des vibrations ultrasonores, le guide d'ondes acoustique ayant une collerette nodale distale (120, 420, 520, 620), et
(ii) une partie distale (206, 800, 902) s'appuyant radialement vers l'intérieur contre la collerette nodale distale, la partie distale coopérant avec la collerette nodale distale pour empêcher un mouvement de rotation de la collerette nodale distale par rapport à la partie distale et pour empêcher un mouvement longitudinal de la collerette nodale distale par rapport à la partie distale ;
(b) un effecteur terminal (16) au niveau d'une extrémité distale de l'ensemble tige, l'effecteur terminal comportant :
(i) une lame à ultrasons (110, 410, 510, 610), la lame à ultrasons étant positionnée au niveau d'une extrémité distale du guide d'ondes acoustique, et
(ii) un bras de serrage (40) fonctionnel pour pivoter vers et à l'écart de la lame à ultrasons,
dans lequel la partie distale comporte un manchon d'armature (200, 800) positionné coaxialement autour de la collerette nodale distale, le manchon d'armature ayant une section de sertissage (206, 800), la section de sertissage étant sertie autour de la collerette nodale distale pour s'appuyer de ce fait radialement vers l'intérieur contre la collerette nodale distale.

2. Instrument chirurgical à ultrasons selon la revendication 1, la collerette nodale distale comportant une pluralité d'évidements (124, 424, 524).

3. Instrument chirurgical à ultrasons selon la revendication 2, les évidements s'étendant longitudinalement à travers un milieu d'une région centrale (122, 522) le long d'une longueur de la collerette nodale distale.

4. Instrument chirurgical à ultrasons selon la revendication 2, les évidements comportant un ensemble distal d'évidements (424) et un ensemble proximal d'évidements (424), l'ensemble distal d'évidements étant positionné distalement par rapport à une partie longitudinalement intermédiaire d'une région centrale (422) le long d'une longueur de la collerette nodale distale, l'ensemble proximal d'évidements étant positionné proximalement par rapport à la partie longitudinalement intermédiaire de la région centrale, la partie longitudinalement intermédiaire de la région centrale fournissant une interruption structurelle entre l'ensemble distal d'évidements et l'ensemble proximal d'évidements.

5. Instrument chirurgical à ultrasons selon l'une quelconque des revendications 2 à 4, chaque évidement de la pluralité d'évidements étant défini au moins en partie par une première surface évidée (126, 426) et une seconde surface évidée (128, 428), la seconde surface évidée fournissant une transition vers la première surface évidée à partir d'autres parties de la collerette nodale distale.

6. Instrument chirurgical à ultrasons selon la revendication 5, la première surface évidée étant plate.

7. Instrument chirurgical à ultrasons selon la revendication 5 ou la revendication 6, la seconde surface évidée étant incurvée.

8. Instrument chirurgical à ultrasons selon l'une quelconque des revendications 2 à 7, chaque évidement ayant une forme concave, facultativement chaque évidement ayant une ayant une surface incurvée définissant la forme concave, et/ou chaque évidement ayant une surface conique négative définissant la forme concave.

9. Instrument chirurgical à ultrasons selon l'une quelconque revendication précédente, la section de sertissage étant malléable.

10. Instrument chirurgical à ultrasons selon l'une quelconque revendication précédente, le bras de serrage étant accouplé de façon pivotante au manchon d'armature.

11. Instrument chirurgical à ultrasons selon l'une quelconque revendication précédente, l'ensemble tige comportant en outre une section d'articulation (80), la section d'articulation étant fonctionnelle pour dévier l'effecteur terminal latéralement à l'écart d'un axe longitudinal central (LA) de l'ensemble tige.

12. Instrument chirurgical à ultrasons selon la revendication 11, le guide d'ondes acoustique comprenant en outre une région flexible s'étendant le long de la section d'articulation, facultativement la collerette nodale distale étant positionnée distale par rapport à la région flexible.

13. Instrument chirurgical à ultrasons selon l'une quelconque revendication précédente, l'ensemble tige comprenant en outre un élément élastomère (300, 700) interposé entre la collerette nodale distale et la partie distale de l'ensemble tige, l'élément élastomère étant comprimé contre la collerette nodale distale par la partie distale de l'ensemble tige.

14. Instrument chirurgical à ultrasons selon la revendication 1, dans lequel l'ensemble tige comprend en outre une section d'articulation (80), la collerette nodale distale étant positionnée distalement par rapport à la section d'articulation, la section d'articulation étant fonctionnelle pour dévier l'effecteur terminal latéralement à l'écart d'un axe longitudinal central de l'ensemble tige.

15. Procédé, comprenant :
(a) le positionnement d'une caractéristique élastomère (300, 700) autour d'une collerette nodale distale (120, 420, 520, 620) d'un guide d'ondes acoustique (100, 400, 500, 600) d'un ensemble tige (14) d'un instrument chirurgical à ultrasons (10) ;
(b) le positionnement d'un manchon malléable (200, 800) autour de la collerette nodale distale de telle sorte que la caractéristique élastomère est radialement interposée entre le manchon malléable et la collerette nodale distale ; et
(c) la compression du manchon malléable radialement vers l'intérieur pour sertir de ce fait le manchon malléable contre la collerette nodale distale et pour comprimer de ce fait la caractéristique élastomère contre la collerette nodale distale, les propriétés malléables du manchon étant conçues pour maintenir le sertissage et la compression, fixant de ce fait fixement le manchon malléable par rapport à la collerette nodale distale.
